# EUROPÄISCHE PATENTSCHRIFT

(11) **EP 0 496 846 B1**
(45) Veröffentlichungstag und Bekanntmachung des Hinweises auf die Patenterteilung: **07.05.1997**
(21) Anmeldenummer: 91913348.8
(22) Anmeldetag: 01.08.1991
(51) Int. Cl.: C12P 33/16

(54) **VERFAHREN ZUR HERSTELLUNG VON 17-OXOSTEROIDEN**
METHOD OF PREPARING 17-OXOSTEROIDS
PROCEDE POUR LA FABRICATION DE 17-OXOSTEROIDES

(30) Priorität: 18.08.1990 DE 4026462
(43) Veröffentlichungstag der Anmeldung: 05.08.1992
(73) Patentinhaber: SCHERING AKTIENGESELLSCHAFT, 13342 Berlin (DE)
(72) Erfinder: WEBER, Alfred, D-1000 Berlin 37 (DE); KENNEKKE, Mario, D-1000 Berlin 33 (DE); KLAGES, Uwe, D-1000 Berlin 28 (DE); NICKISCH, Klaus, D-1000 Berlin 49 (DE); ROHDE, Ralph, D-1000 Berlin 45 (DE)
(86) Internationale Anmeldenummer: DE9100628
(87) Internationale Veröffentlichungsnummer: WO9203572

(56) Entgegenhaltungen:
- EP-A- 129 499
- EP-A- 322 081
- DE-A- 1 568 025

## Beschreibung

Die Erfindung betrifft ein Verfahren zur Herstellung von 17-Oxosteroiden durch fermentative Oxidation von 17β-Hydroxysteroiden.

Verfahren zur fermentativen Herstellung von 17-Oxosteroiden sind seit langem bekannt. Bereits 1938 beschreiben A. Vercellone und L. Momoli ein Verfahren zur Herstellung von 4-Androsten-3,17-dion durch Fermentation von 5-Androsten-3β,17β-diol (Ber. 71, 1938, 152-155). Dieses Verfahren liefert hohe Ausbeuten an Verfahrensprodukt, wenn man die geeigneten Mikroorganismen auswählt (Czech 87.068; ref. C.A. 54, 1960, 2441). Man findet auch sehr viele Publikationen, in denen beschrieben ist, daß man 17-Oxosteroide (wie 4-Androsten-3,17-dion, 1,4-Androstadien-3,17-dion oder 3-Hydroxy-1,3,5(10)-estratrien-17-on) unter Erzielung hoher Ausbeuten durch fermentativen Seitenkettenabbau von Substraten, die in der 17β-Position eine Kohlenwasserstoffkette tragen. herstellen kann.

Aus der DE-A 1 568 025 ist ein Verfahren bekannt, das die fermentative Umsetzung beschreibt, wobei der A-Ring der Steroide jeweils aromatisiert wird.

Über Verfahren, mit deren Hilfe es möglich ist 17β-Hydroxysteroide in guter Ausbeute mikrobiologisch zu 17-Oxysteroiden zu oxidieren, ohne daß eine zusätzliche Umwandlung des Steroides erfolgt, ist nur sehr wenig bekannt. M. Welsh und C. Heusghem beschreiben ein Verfahren, daß es ermöglichen soll. Estradiol mit einer Ausbeute von 95 % in Estron umzuwandeln, aber dieses Verfahren ist nicht nacharbeitbar, da der von diesen Autoren verwendete Mikroorganismus der Offentlichkeit nicht zugänglich ist (Compt. Rend. Soc. Biol., 142, 1948, 1074).

Es besteht aber ein erheblicher Bedarf an der Bereitstellung eines derartigen fermentativen Verfahrens, da die chemischen Verfahren zur Oxidation recht aufwendig sind. Zusätzlich sind diese chemischen Verfahren mit dem Mangel behaftet, daß sie mittels Reagentien wie Pyridin und Schwefeltrioxid durchgeführt werden, deren umweltverträgliche Entsorgung recht problematisch ist.

Es wurde nun gefunden, daß man 17-Oxosteroide überraschenderweise in hohen Ausbeuten aus 17β-Hydroxysteroiden herstellen kann, wenn man zur Fermentation der 17β-Hydroxysteroide Bakterienkulturen der Spezies Mycobacterium spec. NRRL B-3805, Mycobacterium spec. NRRL B-3683, Mycobacterium phlei NRRL B-8154 oder Mycobacterium fortuitum NRRL B-8153 verwendet.

Die Stämme können von der NRRL (ARS Culture Collection, Northern Regional Research Laboratory U.S. Department of Agriculture, Peoria,Illinois, U.S.A.) bezogen werden. Die Stämme NRRL B-3683 und NRRL B-3805 können auch über die DSM (Deutsche Sammlung von Mikroorganismen und Zellkulturen GmbH) unter den Nummern DSM 2966 und DSM 2967 bezogen werden.

Das erfindungsgemäße Verfahren wird unter den gleichen Fermentationsbedingungen durchgeführt, welche man auch bei den bekannten mikrobiologischen Umwandlungen mit diesen Bakterienkulturen verwendet.

Unter den für diese Bakterienkulturen üblicherweise verwendeten Kulturbedingungen werden in einem geeigneten Nährmedium unter Belüften Submerskulturen angezüchtet. Dann setzt man den Kulturen das Substrat (in einem geeigneten Lösungsmittel gelöst oder in emulgierter Form) zu und fermentiert, bis eine maximale Substratumwandlung erreicht ist.

Geeignete Substratlösungsmittel sind beispielsweise Methanol, Ethanol, Glykolmonomethylether, Dimethylformamid oder Dimethylsulfoxyd. Die Emulgierung des Substrats kann beispielsweise bewirkt werden, indem man dieses in mikronisierter Form oder in einem mit Wasser mischbaren Lösungsmittel (wie Hethanol, Ethanol, Aceton, Glykolmonomethylether, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Ethylenoxyaddukte oder Fettsäureester von Polyglykolen. Als geeignete Emulgatoren seien die handelsüblichen Netzmittel Tegin®, Tween® und Span® beispielsmäßig genannt.

Die optimale Substratkonzentration, Substratzugabezeit und Fermentationsdauer ist von der Art des verwendeten Substrates und Mikroorganismus und den Fermentationsbedingungen abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Zur Durchführung des erfindungsgemäßen Verfahrens werden als Substrate vorzugsweise 17β-Hydroxysteroide der allgemeinen Formel I 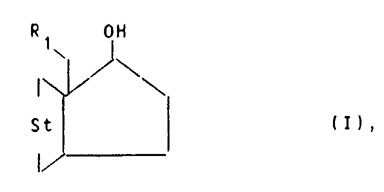 worin
- R₁: ein Wasserstoffatom oder eine Methylgruppe darstellt und
- St: den Rest eines bis zu 5 nicht kummulierte Doppelbindungen enthaltenden, in der 3-Position durch eine phenolische Hydroxygruppe, eine nieder Alkoxygruppe, eine nieder-1-Alkoxy-alkoxygruppe, eine 2-Tetrahydropyranyloxygruppe, oder eine nieder-Alkylendioxygruppe, sowie gegebenenfalls in der 5,10-Position durch eine Epoxygruppe und in der 19-Position gegebenenfalls durch eine Methylgruppe substituierten Estran-17β-ol-Derivates symbolisiert, eingesetzt.

Unter einer nieder-Alkoxygruppe soll vorzugsweise eine Gruppe verstanden werden die bis zu 4 Kohlenstoffatomen trägt. Geeignete Alkoxygruppen sind beispielsweise die Methoxygruppe oder die tert.-Butyloxygruppe. Unter einer nieder-1-Alkoxy-alkoxygruppe soll vorzugsweise eine nieder-Alkoxymethoxygruppe mit einer bis zu 4 Kohlenstoffatomen besitzenden Alkoxygruppe verstanden werden. Beispielsweise genannt sei die 1-Methoxymethoxygruppe. Unter einer nieder-Alkylendioxygruppe soll vorzugsweise eine 2 bis 6 Kohlensoffatome besitzende Gruppierung wie zum Beispiel die Ethylendioxygruppe oder die 2,2-Dimethyl-propylendioxygruppe verstanden werden.

Substrate, die sich zur Durchführung des Erfindungsgemaßen Verfahrens eignen sind beispielsweise Estran-17β-ol-Derivate der allgemeinen Formel Ia 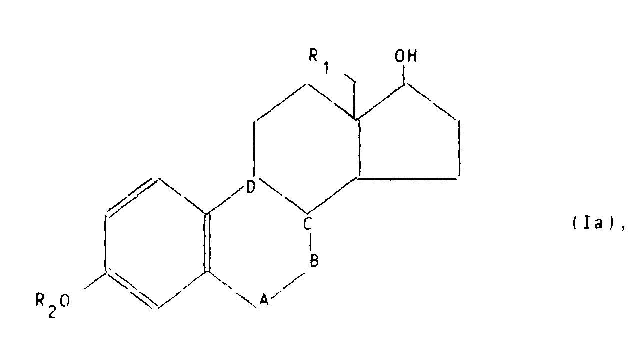 worin
- R₁: die obengenannte Bedeutung besitzt,
- R₂: ein Wasserstoffatom oder eine bis zu 4 Kohlenstoffatome enthaltende Alkylgruppe darstellt und 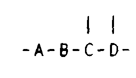 eine Gruppierung der Teilformel 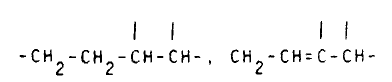 oder 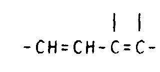 symbolisiert.

Die aus diesen Substraten gebildeten Estran-17-on-Derivate beziehungsweise die hieraus durch Etherspaltung erhaltenen 3-Hydroxyverbindungen sind bekanntlich pharmakologisch wirksam und können darüberhinaus bekanntlich den entsprechenden 17α-Hydroxyverbindungen reduziert werden oder in die entsprechenden 17β-Hydroxy-17α-ethinylverbindungen überfuhrt werden.

Geeignete Substrate sind ferner Estran-Derivate der allgemeinen Formel Ib 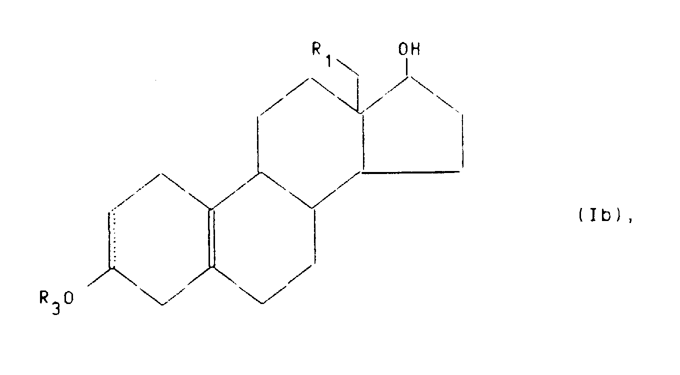 worin
. . . . . eine Einfachbindung oder eine Doppelbindung symbolisiert,
- R₁: die obengenannte Bedeutung besitzt,
- R₃: eine Alkylgruppe mit maximal 4 Kohlenstoffatomen darstellt.

Die aus diesen Verbindungen gebildeten 17-Oxosteroide konnen bekanntlich beispielsweise zur Herstellung des pharmakologisch wirksamen 17α-Ethinyl-17β-hydroxy-4-estren-3-ons beziehungsweise dessen 18-Methylhomologen dienen.

Erwähnenswerte Substrate sind auch solche der allgemeinen Formel Ic 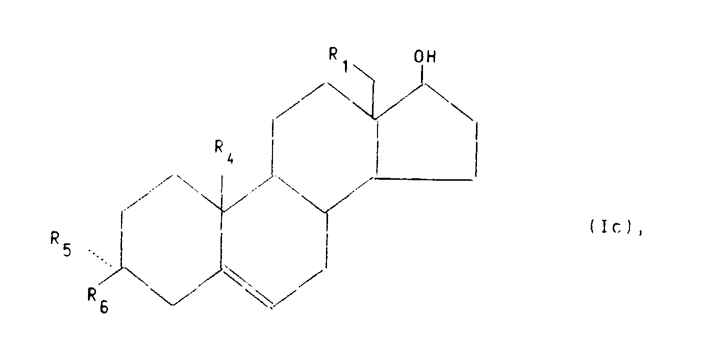 worin
- R₁: die obengenannte Bedeutung besitzt,
- R₄: ein Wasserstoffatom oder eine Methylgruppe darstellt
- R₅ und R₆: gemeinsam eine nieder-Alkyldioxygruppe symbolisieren oder
- R₅: ein Wasserstoffatom bedeutet und
- R₆: eine nieder-Alkoxygruppe, eine nieder-1-Alkoxyalkoxygruppe oder eine 2-Tetrahydropyranylgruppe darstellt.

Die aus diesen Verbindungen erhaltenen 17-Oxosteroide konnen in gleicher Weise weiterverarbeitet werden, wie die aus den Estran-17β-ol-Derivaten der allgemeinen Formel Ic; sie konnen aber andererseits auch zur Herstellung von Pregnan-Derivaten dienen, wie dem Fachmann wohl bekannt ist.

Erwähnenswert sind letztlich auch Estran-17β-ol-Derivate der allgemeinen Formel Id 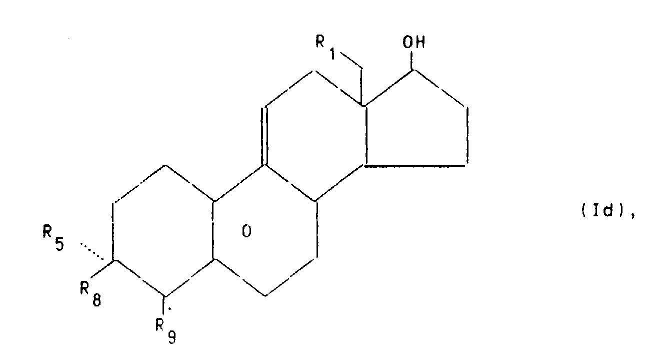 worin
- R₁: die obengenannte Bedeutung besitzt,
- R₉: ein Wasserstoffatom sowie R₇ und R₈ gemeinsam eine nieder-Alkylendioxygruppe symbolisieren oder
- R₇ und R₉: zwei Wasserstoffatome oder gemeinsam eine Kohlenstoff-Kohlenstoff-Bindung bedeuten und
- R₈: eine nieder-Alkoxygruppe, eine nieder-1-Alkoxyalkoxygruppe oder eine 2-Tetrahydropyranyloxygruppe darstellt.

Die aus diesen Verbindungen erhaltenen Ketone können nach dem Verfahren, wie es im EP-A 0129499 beschrieben ist, in antigestagen wirksame Steroide uberführt werden.

Die nachfolgenden Ausführungsbeispiele dienen zur näheren Erläuterung des erfindungsgemäßen Verfahrens.

### Beispiel 1

a) Ein 500 ml Erlenmeyer mit 100 ml sterilem Nahrmedium enthaltend

| | |
|---|---|
| 1 % | Hefeextrakt |
| 0,45 % | Na₂HPO₄ . 2H₂O |
| 0,34 % | KH₂PO₄ |
| 0,2 % | Tween 80 |

mit einem pH von 6,7 wird mit einer Suspension einer Mycobacterium spec. NRRL 8-3683 Kultur beimpft und 72 Stunden bei 30° C mit 180 Umdrehungen pro Minute geschüttelt.
b) 50 Erlenmeyer (100 ml) mit jeweils 20 ml sterilem Nahrmedium enthaltend

| | |
|---|---|
| 0,5 % | Cornsteep liquor |
| 0,05 % | Glucosemonohydrat |
| 0,2 % | Hefeextrakt |
| - eingestellt auf pH 7,0 - | |

werden mit jeweils 1 ml der Mycobacterium-spec.-Anzuchtskultur beimpft und 24 Stunden auf einem Rotationsschuttler mit 220 Umdrehungen pro Minute bei 30° C inkubiert.
Dann setzt man jeder Kultur 0,02 g in 0,2 ml Dimethylformamid gelöstes und sterilfiltriertes 3,3-(2,2-ilmethyltrimethylendioxy)-5,10α-epoxy-5α-estr-9(11)-en-17β-ol zu und fermentiert weitere 48 Stunden bei 30° C.
c) Die vereinigten Kulturen, werden mit Methylisobutylketon extrahiert und der Extrakt im Rotationsverdampfer bei max. 50° C unter Vakuum eingeengt. Anschließend erfolgt eine Reinigung durch Chromatographie über eine Kieslegelsäule.

Man erhält so 0,85 g 3,3-(2,2-Dimethyltrimethylendioxyl-5,10α-epoxy-5α-estr-9(11)-en-17β-on, welches nach HPLC mit einer authentischen Probe identisch ist.

### Beispiel 2

a) Die Herstellung der Anzuchtskultur erfolgt wie im Beispiel 1a) angegeben.
b) 50 Erlenmeyer (100 ml) mit jeweils 20 ml sterilem Nährmedium enthaltend

| | |
|---|---|
| 2,5 % | Cornsteep liquor |
| 0,25 % | Sojamehl |
| 0,3 % | (NH₄)₂HPO₄ |
| 0,25 % | Tween 80 |
| - eingestellt auf pH 6,5 - | |

werden mit jeweils 1 ml der Mycobacterium-spec.-Anzuchtskultur beimpft und 24 Stunden auf einem Rotationsschüttler mit 220 Umdrehungen pro Minute bei 30° C inkubiert.
Dann setzt man jeder Kultur 0,002 g in 0,2 ml Dimethylformamid gelöstes und sterilfiltriertes α-Equilol und fermentiert weitere 72 Stunden bei 30° C.
c) Die Isolierung des Produktes erfolgt wie unter 1c) beschrieben.
Man erhält so 0,095 g Equilin, welches nach HPLC mit einer authentischen Probe identisch ist.

## Patentansprüche

1. Verfahren zur Herstellung von 17-Oxosteroiden durch fermentative Oxidation von 17β-Hydroxysteroiden, dadurch gekennzeichnet, daß man zur Fermentation eine Bakterienkultur der Spezies Mycobacterium spec. NRRL B-3805. Mycobacterium spec. NRRL B-3683, Mycobacterium phlei NRRL B-8154 oder Mycobacterium fortuitum NRRL B-8153 verwendet.

2. Verfahren zur Herstellung von 17-Oxosteroiden durch fermentative Oxidation von 17β-Hydroxysteroiden gemäß Patentanspruch 1, dadurch gekennzeichnet, daß man ein 17β-Hydroxysteroid der allgemeinen Formel I 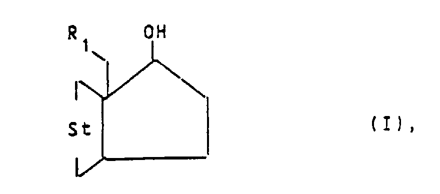 worin
R₁ ein Wasserstoffatom oder eine Methylgruppe darstellt und
St den Rest eines bis zu 5 nicht kummulierte Doppelbindungen enthaltenden, in der 3-Position durch eine phenolische Hydroxygruppe, eine nieder-Alkoxygruppe mit bis zu 4 Kohlenstoffatomen, eine nieder-1-Alkoxy-alkoxygruppe mit nieder-Alkoxymethoxy mit bis zu 4 Kohlenstoffatomen, eine 2-Tetrahydropyranyloxygruppe, oder eine nieder-Alkylendioxygruppe mit einer 2 bis 6 Kohlenstoffatome besitzenden Gruppierung, sowie gegebenenfalls in der 5,10-Position duch eine Epoxygruppe und in der 19-Position gegebenenfalls durch eine Methylgruppe substituierten Estran-17β-ol-Derivates symbolisiert, oxidiert.

## Claims

1. Process for the production of 17-Oxosteroids by fermentative oxidation of 17β-hydroxysteroids, characterized by using a bacterium culture of species Mycobacterium spec. NRRL B-3805, Mycobacterium spec. NRRL B-3683, Mycobacterium phlei NRRL B-8154, or Mycobacterium fortuitum NRRL-B-8153 for the fermentation.

2. Process for the production of 17-Oxosteroids by fermentative oxidation of 17β-hydroxysteroids according to patent claim 1, characterized by oxidizing a 17β-hydroxysteroid of the general formula I 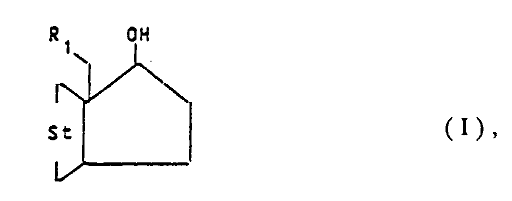 wherein
R₁ represents a hydrogen atom or a methyl group and
St symbolizes the residue of an estran-17β-ol derivative, containing up to 5 not cumulative double bounds, substituted in the third position by a phenolic hydroxy group, a lower alkoxy group having up to 4 carbon atoms, a lower 1-alkoxy-alkoxy group with a lower alkoxy methoxy up to 4 carbon atoms, a 2-tetrahydropyranoyloxy group, or a lower alkylendioxy group with a group consisting of 2 to 6 carbon atoms, as well as optionally an epoxy group in the 5,10 position and optionally a methyl group in position 19.

## Revendications

1. Procédé pour la préparation de 17-oxostéroïdes par oxydation par fermentation des 17β-hydroxystéroïdes caractérisé en ce que l'on utilise pour la fermentation une culture de bactéries des espèces Mycobactérium spec. NRRL B-3805, Mycobacterium spec. NRRL B-3683, Mycobacterium phlei NRRL B-8154 ou Mycobacterium fortuitum NRRL B-8153.

2. Procédé pour la préparation de 17-oxostéroïdes par oxydation par fermentation de 17β-hydroxystéroïdes selon la revendication 1, caractérisé en ce qu'on oxyde un 17β-hydroxystéroïde de formule générale I 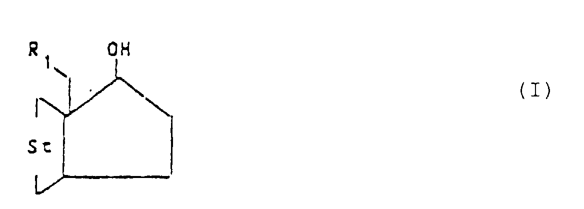 dans laquelle
R¹ représente un atome d'hydrogène ou un groupe méthyle et
St représente un radical d'un dérivé d'estran-17β-diol contenant jusqu'à 5 doubles liaisons non cumulées, substitué en position 3 par un groupe hydroxyle de phénol, un groupe alcoxy inférieur contenant jusqu'à 4 atomes de carbone, un groupe 1-alcoxy-alcoxy inférieur avec alcoxyméthoxy inférieur comportant jusqu'à 4 atomes de carbone, un groupe 2-tétra-hydropyranyloxy ou un groupe alkylènedioxy inférieur avec un groupement comportant de 2 à 6 atomes de carbone, ainsi que substitué éventuellement en la position 5,10 par un groupe époxy et éventuellement en la position 19 par un groupe méthyle.
